Europäisches Patentamt

European Patent Office

Office européen des brevets

(19)

(11) Publication number: 0 378 896
A2

# EUROPEAN PATENT APPLICATION

(21) Application number: 89310037.0

(22) Date of filing: 02.10.89

(51) Int. Cl.5: G01T 1/00, G01T 1/29

(30) Priority: 23.11.88 US 275782

(43) Date of publication of application:
25.07.90 Bulletin 90/30

(84) Designated Contracting States:
DE FR GB NL

(71) Applicant: PICKER INTERNATIONAL, INC.
595 Miner Road
Highland Heights Ohio 44143(US)

(72) Inventor: Mattson, Rodney A.
6532 Melshore Drive
Mentor Ohio 44060(US)

(74) Representative: Pope, Michael Bertram
Wingate
The General Electric Company, p.l.c., GEC
Patent Dept.(Wembley Office), Hirst
Research Centre, East Lane
Wembley Middlesex HA9 7PP(GB)

(54) Radiation detectors.

(57) Radiation detectors (20) of a computed tomography scanner (10) each have a radiation receiving face (34) which is larger than a photosensitive face (30) of a photodiode (22). Lead wires (28) connect the ends of the diode photosensitive surface to terminals (26). A scintillation crystal (32) has an overhanging porfion (38) which overhangs at least the interconnection between the lead wires and the photosensitive face to protect the adjoining areas of the photosensitive face fro incident radiation. This enables the radiation receiving surface to be larger than the photosensitive surface. The crystal is either undercut to define the overhanging area or a section of light pipe (60) is provided between the photosensitive surface and the crystal. Increasing the radiation receiving face decreases rotor ripple artifacts. Decreasing the photosensitive face area decreases diode capacitance and increases resistance which improves amplifier performance.

FIG. 2

## Radiation Detectors

This invention relates to radiation detectors.

The invention finds particular application in conjunction with computed tomography (CT) scanners and will be described with reference thereto. It is to be appreciated, however, that the invention may find further application in other radiation detection devices.

In fourth generation CT scanners, a plurality of x-ray detectors have been stationarily mounted in a ring circumscribing the scan circle. X-ray energy emitted by the x-ray tube was directed to impinge upon a segment of detectors disposed on the opposite side of the scan circle. As the x-ray tube rotated around the scan circle, the irradiated portion of the stationary detector array shifted.

Typically, each detector included a scintillation crystal which converts x-ray energy into light energy. A silicon photodiode converted the light energy into an electric current. The scintillation crystal was normally a rectangular prism which is cut to match the rectangular photosensitive face of the photodiode.

Conventional photodiodes have lead wires connected to opposite ends of their photosensitive face. In order to obtain good optical coupling between the scintillation crystal and the photosensitive face, scintillation crystals terminated short of the lead wire connections. That is, the scintillation crystal has actually been smaller than the photosensitive face. This left a small length of photodiode which was not shielded by the scintillation crystal from the incident radiation. If any x-rays impinged upon the exposed photodiode surface, they tended to induce charges which migrated to the diode collector electrodes and contributed an undesirable component to the diode current.

A preamplifier amplified the photodiode current to produce a voltage signal indicative of the intensity of the radiation incident on the scintillation crystal. Of course, the performance of the CT scanner was dependent on how faithfully these components report the intensity of the incident radiation.

To optimize the results, the preamplifier should be selected such that its output is limited by the x-ray photon flux and not by electronic noise. To achieve this performance goal, preamplifier circuit designs commonly required a low capacitance and high resistance input. Because the capacitance dropped and the resistance increased as the photosensitive area of the photodiode decreased, optimal preamplifier performance called for a small photodiode.

However, other design criteria called for a large scintillation crystal, hence, large photosensitive diode face. More specifically, fourth generation scanners have been sensitive to transverse movement of the x-ray spot. The transverse focal spot movement was commonly due to wobbling of the rotating anode target, anode surface irregularities, or the like. The focal spot wobble caused corresponding periodic fluctuations in the x-ray tube output. These periodic fluctuations caused interference patterns to be superimposed on the CT image, known as "rotor ripple" artifacts. The transverse wobble of the x-ray spot tended to cause a like transverse wobble of the x-ray fan beam, shifting the beam in part off the scintillation crystals of the x-ray detectors. Elongated scintillation crystals enabled the full width of the x-ray beam to be received even during anode target wobble, hence, reduced wobble artifacts. However, elongating the scintillation crystal heretofore required elongating or enlarging the photosensitive diode face which increased its capacitance and reduced its resistance which, in turn, reduced the performance of the preamplifier. Thus, there has been a trade-off between amplifier noise signal degradation and rotor ripple signal degradation.

It is an object of the present invention to provide a radiation detector wherein the above-mentioned problems are overcome.

According to a first aspect of the present invention there is provided a radiation detector for computed tomography scanners comprising: a photodiode having a photosensitive face; and a scintillation crystal having a radiation receiving face and a second face disposed generally parallel to said radiation receiving face and optically coupled to said photosensitive face, characterised in that the area of said radiation receiving face is larger than the area of said photosensitive face.

According to a second aspect of the present invention there is provided a method of fabricating a radiation detector for computed tomography scanners comprising: selecting an area of a photosensitive surface of a photodiode in accordance with preamplifier characteristics; cutting a scintillation crystal to have a radiation receiving surface of an area in accordance with radiation detection characteristics, the area of the radiation receiving surface being larger than the area of the photosensitive surface; undercutting lower edges of the scintillation crystal opposite the radiation receiving surface such that a second surface of the scintillation crystal opposite the radiation receiving surface generally conforms to the photosensitive surface; connecting lead wires to the photosensitive surface adjacent at least one edge of the photosensitive surface; and optically coupling said second surface with said photosensitive surface such that the lead wires are received in the undercut regions of the scintillation crystal and such that the scintillation crystal overhangs the connection between the lead wires and photosensitive surface.

Three radiation detectors in accordance with the present invention will now be described, by way of example, with reference to the accompanying drawings in which:-

Figure 1 is a diagrammatic illustration of a CT scanner that is suitable for using the three detectors;

Figure 2 is a side view of the first detector;

Figure 3 is a plan view of the detector of Figure 2;

Figure 4 is a side view of part of the second detector; and

Figure 5 is a side view of part of the third detector.

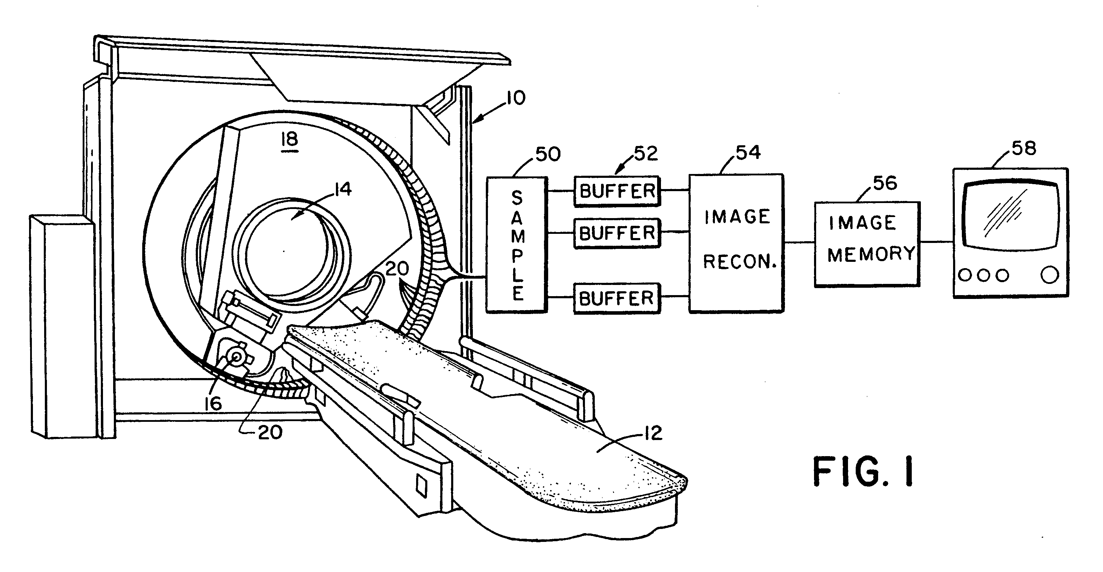

Referring to Figure 1, a CT scanner 10 is mounted in association with a patient supporting couch 12. The couch selectively moves a portion of a patient to be imaged into an examination region or scan circle 14. An x-ray tube 16 15 mounted on a rotatable gantry 18 for selectively rotating a fan beam of radiation around the scan circle. A plurality of radiation detectors 20 are stationarily mounted In a ring surrounding the gantry 18 and the scan circle. The x-ray tube with associated fan beam defining structures, the gantry, and the detectors are mounted such that a planar fan beam of radiation moves along detectors irradiating a contiguous arc or subset of the detectors.

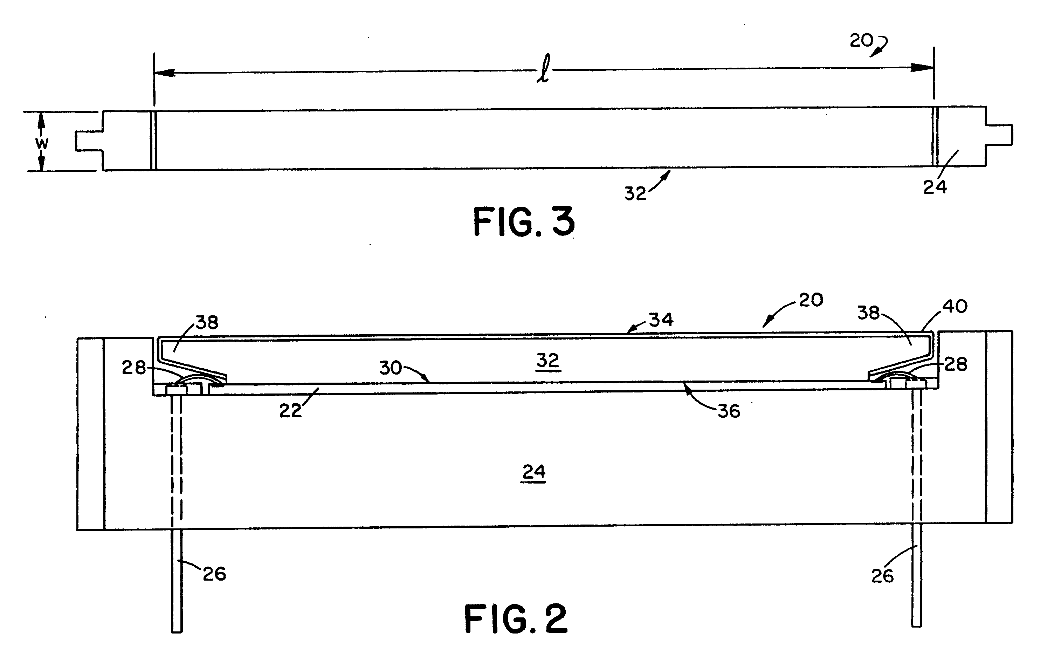

With particular reference to Figures 2 and 3, each first detector includes a photodiode 22 which is supported by a substate or mounting bracket 24. A plurality of electrical contact pins 26 extend from the substrate to plug into matching sockets in the CT scanner. Lead wires 28 interconnect the photodiode with the electrical contact pins 26.

An upper face 30 of the photodiode is photosensitive. The electrical lead wires 28 are connected with the photosensitive face 30, closely adjacent each end. A scintillation crystal 32 is optically coupled with the photodiode 22. More specifically, the scintillation crystal has an upper or radiation receiving face 34 and an oppositely disposed inner or second face 36. The second face 36 is optically coupled, such as with an optical bonding resin, to the photosensitive face of the photodiode. The radiation receiving face 34 has a greater area than the photosensitive face 30. More specifically, a portion of the scintillation crystal is under cut at each end to define overhanging crystal portions 38. Preferably, the overhanging crystal portion is relatively thick to shield the edges of the photosensitive face that extend beyond the coupling with the scintillation crystal from incident radiation.

The overhanging portion and upper surface of the scintillation crystal are coated with a light reflective substance 40, such as white paint, or the like. Incident radiation passes through the light reflective coating on the radiation receiving surface 34 and is converted into light energy by the scintillation crystal. A portion of the light travels directly to the photosensitive face of the diode and the rest of the light travels towards other surfaces of the scintillation crystal. The light reflective surfaces reflect the rest of the light back into the crystal. Some of the reflected light eventually travels or is reflected to the photosensitive face of the diode. By angling the lower surface of the overhanging portion 38 of the scintillation crystal, more of the light from scintillations occurring in the overhanging portion are reflected toward the central portion of the scintillation crystal to improve the chances that the light will hit or be further reflected.

To manufacture the detector, the photodiode is selected or sized to meet capacitive and shunt resistive input specifications of a preamplifier. The preamplifier in tandem with the photodiode is designed to provide an appropriate gain bandwidth, frequency response and noise specifications. The diode specification, such as the area of the photosensitive face, may then be adjusted, as necessary, in order to improve the performance characteristics of the amplifier. The scintillation crystal is cut to the length and width specifications of the CT scanner. The crystal is sized long enough to subtend the full radiation fan beam including the penumbral region associated with an x-ray tube focal spot of finite size, alignment tolerances of the x-ray tube and detector ring, and focal spot wobble. The length of the detector is also selected to be sufficiently longer than the photodiode to overhang the portion of the photodiode face at which the lead wires are connected. Material is removed from the underside of each end of the crystal to eliminate mechanical interference with the lead wires. The undercut can be a bevel, chamfer, a small rectangular step, or the like. Surfaces of the crystal, except for the diode coupling face 35 but including the undercut area, are coated with white paint or other reflective material 40. The second crystal face is optically bonded to the active area of the photosensitive face between the lead wires and the crystal, photodiodes, and substrate are epoxy bonded together.

It is to be noted that although the reflective coating on the overhanging portion reflects light back into the volume of the crystal, that light must be reflected at least once more before hitting the photodiode. Accordingly, radiation impacting the overhanging portion will produce a subdued response in the output signal.

With reference again to Figure 1, the output signals from the amplifier associated with each photodetector are sampled by a sampling means 50. Buffer memories 52 may store the sampled. data temporarily prior to image reconstruction by an image reconstructing means 54. An image memory 56 stores the reconstructed image which may be displayed on a video monitor 58, stored in computer memory or on tape, subject to further processing, or the like.

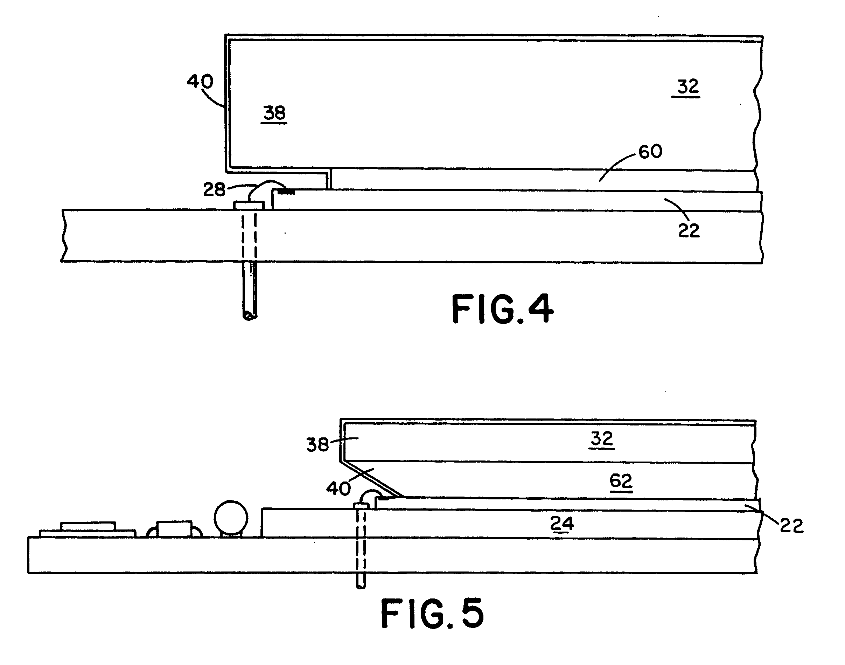

Referring to Figure 4, in the second detector to be described the scintillation crystal 32 is again larger than the photosensitive face of the diode 22. However, rather than cutting the crystal, an optic light pipe or guide 60 is connected between the photosensitive face 30 and the crystal second face 36. The light guide extends perpendicular to the photosensitive face for a distance sufficient for the crystal lower face to clear the lead wires. Overhanging portions 38 are again coated with the light reflective material 40. The exterior surface around the periphery of the light guide is also coated with the light reflective material. Alternately, the light guide may be a scintillation crystal that coverts radiation passing through the upper scintillation crystal into light.

In the third detector to be described shown in Figure 5, the scintillation crystal 32 is again larger than the photosensitive face 30 of the diode. A tapered section 62 of light transmissive material has the same upper cross section as the lower surface of the crystal and the same lower cross section as the photosensitive face. The light transmissive material may be a light guide or may be an additional piece of scintillation crystal to increase the x-ray capture capacity of the detector. The lower scintillation crystal may be the same crystalline substance as the upper scintillation crystal or may be a different scintillator, such as a scintillator with greater x-ray stopping power, one which produces light of a different spectrum, or the like.

## Claims

1. A radiation detector for computed tomography scanners comprising: a photodiode (22) having a photosensitive face (30); and a scintillation crystal (32) having a radiation receiving face (34) and a second face (36) disposed generally parallel to said radiation receiving face (34) and optically coupled to said photosensitive face (30), characterised in that the area of said radiation receiving face (34) is larger than the area of said photosensitive face (30).

2. A detector according to Claim 1 wherein said photosensitive face (30) has a first length and a first width, said second face (36) has a second length and a second width and said radiation receiving face (34) has a third length (l) and a third width (w), the third length (l) being longer than the first length.

3. A detector according to Claim 2 further including a lead wire (28) connected with said photosensitive face (30) adjacent one end of said photosensitive face (30) and wherein the third length (l) is longer than the first length by a sufficient distance so that a portion (38) of the scintillation crystal (32) overhangs the connection of the lead wire (28) with said photosensitive face (30).

4. A detector according to Claim 1 or Claim 2 or Claim 3 further including an optic light pipe (60) interconnecting said second face (36) with said photosensitive face (30).

5. A detector according to Claim 3 wherein the scintillation crystal (32) has a cut-out portion such that the second length is shorter than the first length by at least a length of the connection of the lead wire (28) with said photosensitive face (30), whereby a portion (38) of the crystal (32) overhangs and shields the connection of the lead wire (28) with said photosensitive face (30).

6. A detector according to Claim 3 or Claim 5 wherein a surface of said overhanging portion (38) adjacent said photosensitive face (30) has a light reflective coating (40).

7. A detector according to Claim 1 or Claim 2 wherein the scintillation crystal (32) has a portion (38) which overhangs lead wires (28) connected to at least one edge of the photodiode (22), a surface of said portion (38) having a light reflective coating (40).

8. A detector according to Claim 1 wherein lead wires (28) are connected adjacent at least one end of said photosensitive face (30), said second face (36) being connected only with an area of said photosensitive face (30) away from the connections of the lead wires (28) to inhibit mechanical interference between the scintillation crystal (32) and the lead wires (28).

9. A detector according to Claim 8 wherein the scintillation crystal (32) has a portion (38) which overhangs the lead wires (28) and is displaced therefrom.

10. A detector according to Claim 9 further including a coating of light reflective material on at least an undersurface of the overhanging portion (38).

11. A detector according to Claim 8 further including an optic light pipe section (60) extending between said second face (36) and said photosensitive face (30), the optic light pipe section (60) being configured such that a portion (38) of the scintillation crystal (32) is displaced from and overhangs the lead wires (28).

12. A detector according to Claim 11 further including a coating (40) of light reflective material on at least an undersurface of the overhanging portion (38).

13. A detector according to Claim 8 wherein the scintillation crystal (32) has a dimension (l) sufficiently larger than a dimension of said photosensitive face (30) that the area of said photosensitive face (30) around the lead wire (28) connections is shielded by the crystal (32) from direct receipt of radiation.

14. A medical diagnostic scanner comprising: a source of radiation (16) for projecting radiation across an examination region (14); and a plurality of radiation detectors (20) according to any one of the preceding claims disposed across the examination region (14) from the source of radiation (16) for receiving radiation therefrom.

15. A method of fabricating a radiation detector for computed tomography scanners comprising: selecting an area of a photosensitive surface (30) of a photodiode (22) in accordance with preamplifier characteristics; cutting a scintillation crystal (32) to have a radiation receiving surface (34) of an area in accordance with radiation detection characteristics, the area of the radiation receiving surface (34) being larger than the area of the photosensitive surface (30); undercutting lower edges of the scintillation crystal (32) opposite the radiation receiving surface (34) such that a second surface (36) of the scintillation crystal (32) opposite the radiation receiving surface (34) generally conforms to the photosensitive surface (30); connecting lead wires (28) to the photosensitive surface (30) adjacent at least one edge of the photosensitive surface (30); and optically coupling said second surface (36) with said photosensitive surface (30) such that the lead wires (28) are received in the undercut regions of the scintillation crystal (32) and such that the scintillation crystal (32) overhangs the connection between the lead wires (28) and photosensitive surface (30).

16. A method according to Claim 15 further including coating at least the overhanging portion (38) of the scintillation crystal (32) with a light reflective material (40).

FIG. I

EP 0 378 896 A2

EP 0 378 896 A2

FIG. 3

FIG. 2

EP 0 378 896 A2

40  38  32  60  28  22

## FIG.4

38  32  62  40  24  22

## FIG.5